# EUROPÄISCHE PATENTSCHRIFT

(11) **EP 3 033 446 B1**
(45) Veröffentlichungstag und Bekanntmachung des Hinweises auf die Patenterteilung: **22.03.2017**
(21) Anmeldenummer: 14758500.4
(22) Anmeldetag: 14.08.2014
(51) Int. Cl.: D01B 1/16, D01B 9/00

(54) **VORRICHTUNG UND VERFAHREN ZUR ISOLIERUNG VON BASTRINDE UND HOLZKÖRPER AUS EINEM BASTPFLANZENSTÄNGEL**
DEVICE AND METHOD FOR ISOLATING BAST BARK AND WOOD BODIES FROM A BAST-PLANT STEM
DISPOSITIF ET PROCÉDÉ PERMETTANT D'ISOLER UNE ÉCORCE LIBÉRIENNE ET UN CORPS EN BOIS ISSUS D'UNE TIGE DE PLANTE LIBÉRIENNE

(30) Priorität: 16.08.2013 DE 102013013657
(43) Veröffentlichungstag der Anmeldung: 22.06.2016
(73) Patentinhaber: Bast & Faser GmbH, 91325 Adelsdorf (DE)
(72) Erfinder: PAULITZ, Jürgen, 01259 Dresden (DE); HERTEL, Robert, 91074 Herzogenaurach (DE); NOWOTNY, Rainer, 17291 Prenzlau (DE)
(74) Vertreter: Meyer, Rudolf
(86) Internationale Anmeldenummer: PCT/EP2014/002237
(87) Internationale Veröffentlichungsnummer: WO 2015/022078

(56) Entgegenhaltungen:
- DE-C- 388 277
- DE-C- 685 925
- DE-C- 754 085

## Beschreibung

Die Erfindung betrifft ein Verfahren zur Isolierung von Bastrinde und Holzkörper aus einem oder mehreren Bastpflanzenstängeln, wobei der Bastpflanzenstängel in seinem Inneren aus einem im Wesentlichen zylindrischen Holzkörper gebildet ist und der Holzkörper radial von einer Bastfasern enthaltenden Bastrinde umfasst wird und über ein Teilungsgewebe mit der Bastrinde verbunden ist. Des Weiteren betrifft die Erfindung eine zugehörige Vorrichtung ein Bastrindenprodukt, ein Bastfaserprodukt und ein Bastpflanzenprodukt.

### Technisches Gebiet

Aus dem Stand der Technik sind verschiedene Aufbereitungsmethoden von Bastfaserpflanzen bekannt. Die aus Bastpflanzen, wie zum Beispiel Flachs, Hanf, Kenaf, Öllein oder Jute, gewonnenen Bastfasern bilden Rohstoffe für unterschiedlichste Anwendungen. Derartige Bastpflanzen können Textilrohstoffe, Werkstoffe und Zwischenprodukte hervorbringen. Bei den Industrierohstoffen unterscheidet man faserbasierte Rohstoffe und schäbenbasierte Rohstoffe. Hierbei richtet sich die Anwendung oder der Einsatz des Rohstoffes nach Eigenschaften, die entweder bei den Fasern, beziehungsweise bei den Schäben zu suchen sind. Die Schäben stammen aus dem holzigen Mark der Bastpflanzenstängel und werden meist beim Zerkleinern des Stängelmarks gebildet. Anschließend werden in verschiedenen bekannten Verfahren Schäben und Fasern getrennt.

DE-C-685,925 zeigt einen bekannten Stand der Technik

Die Verarbeitungskette von Bastpflanzen beginnt mit deren Trennung vom Standort und setzt sich über weitere Feldoperationen bis hin zur Bündelung oder Ballung der geernteten Bastpflanzen fort. In dieser ersten Phase war und ist die manuelle Ausführung notwendiger Operationen eine gangbare Alternative.
Nach einem Transport und gegebenenfalls einer oder mehreren Zwischenlagerungen findet meist eine stationäre Aufbereitung statt, die unter Nutzung mechanischer und hydrothermischer Prozesse ausgeführt wird. Auf diese Verfahren wird nachfolgend detaillierter eingegangen.
Eine weiterführende stationäre Aufbereitung kann durch chemische, bakterielle oder enzymatische Prozessschritte erfolgen. Hierbei wird die Faser für den nachfolgenden Einsatz weiter konditioniert und von den übrigen Bestandteilen der Faserbastrinde getrennt. Anschließend kann ein stationäres Finishing erfolgen, welches das Waschen, Spülen und Trocknen der isolierten Bastfasern einschließen kann.
Bisher wurden Bastfasern beispielsweise für die Garnherstellung verwendet, die hauptsächlich für Bekleidungsprodukte relevant ist. Bastfasern werden auch für Faserverbundwerkstoffe eingesetzt, wobei übliche Kunstfasern ersetzt werden können. Wesentliche Verarbeitungsverfahren zur Herstellung von Formpressteilen basieren auf Faservliesen. Bastfasern werden somit in weiteren industriellen Anwendungsgebieten immer mehr genutzt, wie zum Beispiel im Fahrzeugbau und in der Baustoffindustrie.
An bisherigen Aufschlussverfahren für Bastfaserpflanzen ist problematisch, dass die Qualität der resultierenden Fasern und Schaben beeinträchtigt wird. Die angewendeten Verfahren begrenzen somit den Einsatzbereich von Fasern und Schäben für anspruchsvolle Anwendungen erheblich. So sind unterschiedliche Verfahren zur Fasergewinnung in Abhängigkeit von deren Länge oder von deren Trocknungsgrad bekannt.

Beispielsweise bezieht sich die DE 199 18 166 A1 auf eine Vorrichtung zur Gewinnung von Kurzfasern, die aus getrockneten, vorzerkleinerten Bastfasergewächsen gewonnen werden. Die DE 195 18 188 A1 begrenzt sich ebenso auf die Gewinnung von Kurzfasern. Derartige Kurzfasern eignen sich beispielsweise nicht für die effiziente Herstellung feiner Garne. Stattdessen sind Einsatzgebiete für Produkte wie zum Beispiel Vliese, Formteile oder dergleichen möglich. Des Weiteren sind die bereits bekannten mechanischen Verfahren zur Aufbereitung der Bastfaserpflanzen dadurch beschränkt, dass eine Trocknung des Materials herbeigeführt werden muss oder das Material alternativ in einer anderen Art und Weise vor der Verarbeitung konditioniert werden muss.

Der Einsatz der aus den Bastfasern produzierten Bastfaserprodukte wird weiter beschränkt durch die Art der mechanischen Aufbereitung. Insbesondere die weit verbreiteten Prallaufschlussverfahren führen zu einer nachhaltigen Schädigung der Bastfasern, die oft mit einer Kürzung einhergehend, senkrecht zum Faserverlauf druckgeschädigt werden. Dies geschieht meistens durch Kanten oder Schneideelemente von Rollen oder Walzen, die dazu führen, dass die Pflanzenzellen der in der Bastfaser enthaltenen Fibrillen geschädigt werden. Dabei gehen viele vorteilhafte Eigenschaften wie beispielsweise eine hohe Zugbelastbarkeit unwiederbringlich verloren. Derartige Prallverfahren sind aus der DE 196 26 557 A1, der EP 1 155 172 B1 und der WO 2012/006118 A2 bekannt. Diesen Prallaufschlussverfahren ist meist zu eigen, dass die Stängel der Bastfaserpflanzen der Maschine zugeführt werden und gleichzeitig Prozesse wie Zerkleinern, Zerfasern und Trennen der Fasern von den nichtfaserigen Bestandteilen ausgeführt werden.

Gerade die vorgenannten Prallverfahren führen zu einem relativ hohen Anteil von holzigen Bestandteilen. Im den darauf folgenden Prozessschritten ist es nahezu unmöglich, sämtliche Schäben aus dem Stoffstrom zu eliminieren. Verschiedene Verfahren aus dem Stand der Technik haben sich dieser Problematik gewidmet, wobei insbesondere die DE 101 62 361 A1 von einem erreichbaren Staub- und Schäbengehalt von bestenfalls 3% berichtet. Ebenfalls nachteilig ist bei den Prallverfahren, dass Fettrückstände und auch Fremdkörper in den Stoffstrom gelangen können. Diese Problematik wird in der vorgenannten Schrift ebenfalls thematisiert, so auch in der DE 100 07 509 A1.

Aus dem Artikel "Flachs und Hanf" von Gustav Schaefer aus der Ciba-Rundschau. - Basel, 1944, S. 2262-2294 ist das sogenannte Flachsschleißen bekannt, bei dem eine mühsame, manuelle Schälung von Hanfstängeln beschrieben ist.

### Zusammenfassung der Erfindung

Der Erfindung liegt die Aufgabe zu Grunde, hochwertige Pflanzenfasern zu gewinnen, die in vielen Anwendungsbereichen eingesetzt werden können, wobei die vorgenannten Nachteile eliminiert oder zumindest größten Teils reduziert werden.

Diese Aufgabe wird durch ein Verfahren zur Isolierung von Bastrinde und Holzkörper aus einem Bastpflanzenstängel mit den Merkmalen des Anspruchs 1 gelöst. Ferner wird die Aufgabe durch eine Vorrichtung mit den Merkmalen des Anspruchs 13 gelöst. Ebenso lösen das Bastrindenprodukt mit den Merkmalen des Anspruchs 19 das Bastfaserprodukt mit den Merkmalen des Anspruchs 26 und das Bastpflanzenprodukt mit den Merkmalen des Anspruchs 30 die Aufgabe der Erfindung.

Der Erfindung liegt die Erkenntnis zu Grunde, dass die bisher angewendeten Prallverfahren den Pflanzenrohstoff derart schädigen, dass eine oftmals erforderliche Faserqualität nicht mehr gewährleistet werden kann.

Des Weiteren liegt der Erfindung die Erkenntnis zu Grunde, dass das Wachstumsschicht (Kambium), welches die Faserbastrinde mit dem Holzkörper (Mark) verbindet, zur Unterstützung des Isolationsvorganges vorteilhaft einsetzbar ist.

Erfindungsgemäß ist das Verfahren zur Isolierung von Bastrinde und Holzkörper aus einem Bastpflanzenstängel auch auf eine Vielzahl von Bastpflanzenstängeln anwendbar. Bei diesem Verfahren ist der, beziehungsweise sind die Bastpflanzenstängel im Inneren aus einem im Wesentlichen zylindrischen Holzkörper gebildet. Dieser Holzkörper wird auch Xylem genannt und kann gegebenenfalls einen Hohlraum umschließen, der auch Markparenchym genannt wird. Der Holzkörper wird radial vom Kambium umschlossen, wobei das Kambium das erfindungsgemäße Teilungsgewebe bildet und den Holzkörper mit der Bastrinde verbindet. Anders ausgedrückt, das Kambium trennt die Bastrinde vom Holzkörper. Die Bastrinde hat verschiedene Bestandteile, wie z.B. die Bastfasern, die innerhalb der Bastrinde in Bündeln zusammengefasst sind. Gegebenenfalls weist der Bastpflanzenstängel auch eine Außenhaut (Epidermis) auf, die nochmals um die Faserbündel, beziehungsweise die Bastrinde herum angeordnet ist. Die Außenhaut hat üblicherweise die Funktion, die Bastpflanze nach außen hin zu schützen. Das Kambium stellt den wachsenden Teil der Pflanze dar, der radial nach innen verholzt und radial nach außen Faserbündel, beziehungsweise Bastfasern in der Bastrinde bildet.

Erfindungsgemäß wird die Bastrinde mittels einer Zugkraft entlang der in der Bastrinde verlaufenden Bastfasern vom Holzkörper oder zumindest von einem Bestandteil des Holzkörpers im Bereich des Teilungsgewebes getrennt, wobei ein Trennmittel zwischen die Bastrinde und den Holzkörper oder zwischen die Bastrinde und einen Holzbruchkörper des Holzkörpers eingebracht wird. Ausschlaggebend ist, dass die Zugkraft entlang der in der Bastrinde verlaufenden Bastfasern verläuft. Dies ist vorteilhaft, weil die Bastfasern gerade in dieser Richtung besonders widerstandsfähig sind und hohe Lasten halten oder Kräfte weiterleiten können ohne selbst Schaden zu nehmen. Durch die Wahl der Richtung der Zugkraft wird eine Kräfteaufteilung festgelegt, die zum einen den Holzköper bewegen kann und zum anderen die Bastrinde mit den Bastfasern vom Holzkörper ablöst. Hierbei wird das Teilungsgewebe als schwächste Schicht soweit belastet, dass dieses aufreißt oder zu Teilen aufgespalten wird, wobei sowohl am Holzkörper, als auch an der Bastrinde Reste des Teilungsgewebes haften bleiben können. Die Zugkraftrichtung kann nun so gewählt werden, dass die bevorzugte Kräftezerlegung durch die Winkelwahl eingestellt wird und vorteilhafterweise ein oder mehrere Fördermittel ersetzt. Wichtig ist auch, dass bei diesem Verfahren keine Druck- oder Prallkräfte senkrecht zu den Bastfasern auftreten, die Bastfaserzellen beschädigen könnten. Vorteilhafterweise werden somit impactfrei hochwertige Fasern enthaltende Bastrinde mit reproduzierbaren Qualitätseigenschaften gewonnen. Außerdem kann dabei Stängelmark in Form des unzerstörten Holzkörpers bereitgestellt werden.

Vorteilhafterweise eignet sich das Verfahren sowohl für erntefrische, getrocknete, geröstete oder wiederbefeuchtete Bastpflanzenstängel. Insbesondere können die für das Verfahren verwendeten Bastpflanzenstängel getrocknete oder wiederbefeuchtete Bastpflanzenstängel sein, die bisher kaum oder gar nicht zu bearbeiten waren. In Kenntnis der Erfindung, muss lediglich die Stärke der Zugkraft festgelegt werden, um die unterschiedlich feste Bindung der Bastrinde an den Bastpflanzenstängel zu überwinden. Die mechanischen Eigenschaften der einzelnen Bastpflanzenstängel können mittels der Werte unterschiedlicher Kenngrößen, wie zum Beispiel Biegebruchfestigkeit des Holzkörpers, Haft- und Schälfestigkeit zwischen der Bastrinde und dem Holzkörper, Zugfestigkeit der Bastrinde, Spaltfestigkeit des Holzkörpers und der Bastrinde sowie deren Elastizitätsmoduli, charakterisiert werden. Die genannten Kennwerte können zu einem mechanischen Isolationswiderstandsindex (MIWI) zusammengefasst werden, mit dem es möglich ist, Aussagen über die Ausführbarkeit des erfindungsgemäßen Verfahrens zu treffen.
Durch die Erfindung wird die Trockenverarbeitung überhaupt erst möglich, das heißt, die Isolation der Bastrinde vom getrockneten oder gelagerten Pflanzenstängel ist nun machbar. Bisher musste die Bastpflanze erntefrisch isoliert werden, womit stets ein erheblicher Aufwand verbunden war, zumal alle anfallenden Bastpflanzenbündel vor Ort (auf dem Feld) in kürzester Zeit bearbeitet werden mussten. Da aber nun eine Trockenverarbeitung möglich ist, ist der maschinelle Aufwand deutlich geringer, weil eine Maschine mit getrockneten Pflanzenstängeln zeitlich besser ausgelastet werden kann und somit auch weniger Maschinen erforderlich sind.
Um im erfindungsmäßigen Verfahren unmittelbar nach erfolgtem Knick-Bruch ein selbständiges / prozessinitiiertes Abschälen der Bastrinde vom Holzkörper an zumindest einem der beim Knick-Bruch entstandenen Teilstängel sicherzustellen, ist daher erforderlich, dass der Holzkörper in diesem Bereich des Pflanzenstängels eine derartige Biegesteifigkeit aufweist, um in Abhängigkeit von der jeweiligen Haftfestigkeit zwischen der Bastrinde und dem Holzkörper das Abschälen der Bastrinde auf einer solchen Länge vom Holzkörpers zu gewährleisten, die eine derart große Fläche aufspannt, die das anschließende Einbringen eines Trennmittels in den Bereich zwischen Bastrinde und Holzkörper zur Ausführung des weiteren Schälprozesses ermöglicht. Die Biegesteifigkeit des Holzkörpers ergibt sich dabei aus dem Quotient von Bruchlast und das durch Form sowie Querschnittsfläche des Holzkörpers im betreffenden Abschnitt des Bastpflanzenstängels bestimmbare Widerstandsmoment.

Des Weiteren können mittels dieser Methode bei Verwendung von MIWI-Kennwerten und bei Berücksichtigung weiterer Rahmenbedingungen sowohl die notwendige Bruchlast als auch die erforderliche Zugkraft bestimmt werden, die die Trennung der Bastrinde vom Holzkörper bewerkstelligt, wobei das Teilungsgewebe geteilt wird.

Vorteilhafterweise wird die Zugkraft derart auf die Bastrinde übertragen, dass die Bastrinde einen Winkel zur Bewegungsrichtung oder Längsachse des Hohlkörpers oder zur Längsachse eines zum Hohlkörper gehörenden Bestandteils aufspannt, von dem die Bastrinde abgetrennt wird. Es ist denkbar, dass der Holzkörper während des erfindungsgemäßen Verfahrens weiter zerkleinert wird, wobei erst nach dem Zerkleinern die Bastrinde erfindungsgemäß abgetrennt wird. Grundsätzlich sind Mehrfachteilungen des Holzkörpers zu vermeiden, da ansonsten ähnliche Probleme auftreten können, wie sie bereits aus dem Stand der Technik bekannt sind. Jedoch kann es sein, dass längs gespaltene Stängelteile die Anwendung des Verfahrens unterstützen, von daher können Stängelteile ebenfalls mit dem erfindungsgemäßen Verfahren behandelt werden.

Der Winkel zur Bewegungsrichtung oder zur Längsachse des Holzkörpers wird beispielsweise möglichst senkrecht zur Längsachse des Holzkörpers oder zur Längsachse eines zum Holzkörper gehörenden Bestandteils gewählt, sodass der hauptsächliche Anteil der Zugkraft für die Trennung der Bastrinde vom Holzkörper eingesetzt werden kann. Je kleiner der Winkel gewählt wird, desto höher ist die in den Stängelteil oder dessen Bestandteile eingeleitete Zugkraftkomponente, sodass diese zur Bewegung genutzt werden kann.

Das erfindungsgemäße Verfahren beinhaltet die Schritte:
a. Herstellung eines ersten und zweiten Stängelteils, mittels einer Teilung des Bastpflanzenstängels entlang der Längsachse des Bastpflanzenstängels, wobei der erste Stängelteil einen ersten geschnittenen Holzkörper und der zweite Stängelteil einen zweiten geschnittenen Holzkörper aufweist, und
b. einer Biegung wenigstens des ersten Stängelteils, wobei die Bastrinde des ersten Stängelteils an einer gestauchten Stelle angeordnet ist und die Biegekraft so lange erhöht wird, bis der erste geschnittene Holzkörper auf einer der gestauchten Stelle gegenüberliegenden, gedehnten Stelle des Stängelteils von der Bastrinde weg, derart nach außen aufbricht, dass aus dem ersten geschnittenen Holzkörper ein erster Holzbruchkörper und ein zweiter Holzbruchkörper gebildet werden, und
c. der Trennung der Bastrinde vom ersten Holzbruchkörper.

Das erfindungsgemäße Verfahren kann vereinfachend Knick-Bruch-Isolierung genannt werden. Sowohl die Längsachse des Holzkörpers, als auch die Längsachse eines Bestandteils des Holzkörpers beziehen sich auf die im Wesentlichen zylindrische Form des Holzkörpers, der eine Längsachse aufweist, welche auch mit der Richtung des Pflanzenwachstums übereinstimmt.

Der Begriff Stängelteilung bezieht sich auf dessen Längsteilung in wenigstens zwei Bestandteile und ist somit von der Teilung des Teilungsgewebes zu unterscheiden. Der erste Stängelteil und der zweite Stängelteil können hälftig geteilte Stängelteile sein, die durch eine Teilung entlang der Längsachse entstanden sind. Dies ist jedoch nicht zwingend erforderlich, wenn man die Stängelteilung in einer Ebene ausführt, in der die zentrische Längsachse des Holzkörpers nicht angeordnet ist, sondern zu besagter Ebene parallel verläuft. In jedem Fall muss bei der Stängelteilung der Holzkörper des Stängelteils durchschnitten werden, gleichzeitig werden auch alle weiteren vorhandenen Schichten durchtrennt, wie zum Beispiel das Teilungsgewebe und die Bastrinde.

Die Biegung kann sowohl mit dem ersten, als auch dem zweiten oder beiden Stängelteilen ausgeführt werden. Aufgrund der radialen Ausdehnung des ersten Stängelteils an der Biegestelle entstehen eine gestauchte Stelle und eine der gestauchten Stelle gegenüberliegende, gedehnte Stelle. Der erste Stängelteil wird nun derart orientiert, dass die Bastrinde an der gestauchten Stelle und der freigelegte Holzkörper auf der gedehnten Stelle des Stängelteils angeordnet sind. Die Biegung wird so lange ausgeführt, beziehungsweise die Biegekraft wird so lange erhöht, bis der erste geschnittene Holzkörper des ersten Stängelteils derart nach außen aufbricht, dass aus dem ersten geschnittenen Holzkörper ein erster Holzbruchkörper und ein zweiter Holzbruchkörper gebildet werden. Verfahrensrelevant ist, dass die Bastrinde bei der Biegung und beim Bruch unbeschädigt bleibt, weil die Belastung an der gestauchten Stelle ebenfalls hauptsächlich vom ersten geschnittenen Holzkörper aufgenommen wird. Der erste geschnittene Holzkörper hingegen bricht auf, wobei dieser Bruch ein vollständiger Bruch oder nur ein Teilbruch sein kann. Bei einem vollständigen Bruch sind die Holzbruchkörper vollständig voneinander separiert, wobei bei einem Teilbruch noch Teile des Holzkörpers Verbindungen zwischen dem ersten Holzbruchkörper und dem zweiten Holzbruchkörper bilden. Ausschlaggebend ist, dass die Bastrinde dabei ausreichend freigelegt ist und somit vom ersten Holzbruchkörper und idealerweise auch vom zweiten Holzbruchkörper getrennt werden kann. Daran ist vorteilhaft, dass die Trennung und die Biegung quasi in einem Arbeitsschritt erfolgen und die Biegemittel auch zur Trennung der Bastrinde eingesetzt werden können.

Vorteilhafterweise ist die Biegung des ersten Stängelteils und gegebenenfalls des zweiten Stängelteils eine Dreipunktbiegung oder eine Vierpunktbiegung. Die jeweilige Biegung bezieht sich darauf, wie viele Biegepunkte für die jeweilige Biegung und den anschließenden Bruch erforderlich sind.

Vorteilhafterweise berührt der erste geschnittene Holzkörper des ersten Stängelteils wenigstens zwei äußere Biegepunkte und die Bastrinde des ersten Stängelteils wenigstens einen inneren Biegepunkt, insbesondere zwei innere Biegepunkte. Sowohl bei der Dreipunktbiegung, als auch bei der Vierpunktbiegung findet eine Relativbewegung statt, wobei es ausreichend ist, dass ein äußerer oder ein innerer Biegepunkt gegenüber mindestens einem anderen Biegepunkt, der ein innerer oder ein äußerer Biegepunkt sein kann, bewegt wird. Relativ bedeutet in diesem Zusammenhang, dass die Position und die Bewegung der Biegepunkte in Bezug zueinander relevant sind, jedoch nicht in Bezug zu einer Isolationsvorrichtung oder einer Insolationsmaschine. Es ist also auch denkbar, dass sich ein Biegepunkt, eine Auswahl von Biegepunkten oder auch alle Biegepunkte in Bezug zu der genannten Vorrichtung bewegen.

Wenigstens einer der inneren und äußeren Biegepunkte kann durch einen Bolzen, eine Stange oder dergleichen ausgeführt sein. So ist beispielsweise auch denkbar, dass lediglich eine Kante eines Vorrichtungsbestandteils ausreicht, um in Kontakt mit dem ersten und/oder zweiten geschnittenen Holzkörper als ein Biegepunkt zu fungieren. Es sei bemerkt, dass ein Biegepunkt in der Realität sicherlich durch eine Auflagefläche realisiert ist, wobei die Größe der Fläche von der Biegekraft abhängt, das heißt, wie stark der zu biegende geschnittene Holzkörper auf den Bolzen, die Stange oder dergleichen gedrückt wird.

Bei einer vorteilhaften Ausführungsform führt wenigstens ein innerer Biegepunkt oder wenigstens ein äußerer Biegepunkt eine Relativbewegung zu einem anderen inneren oder äußeren Biegepunkt aus. Auf diese Weise ist es möglich, mit einer einzigen Bewegung des einen äußeren oder des einen inneren Biegepunktes einen erfindungsgemäßen Bruch des Holzkörpers herbeizuführen, wobei der jeweils einzige innere oder einzige äußere Biegepunkt auch zu einem Bruchpunkt wird. Dies bedeutet, dass der Bruchbereich in der Nähe dieses einzigen inneren oder einzigen äußeren bewegbaren Biegepunktes angesiedelt ist. Entsprechend vorteilhaft ist es, wenn wenigstens ein innerer Biegepunkt oder wenigstens ein äußerer Biegepunkt eine Relativbewegung zu einem anderen inneren oder äußeren Biegepunkt ausführen kann.

Vorteilhafterweise ist der Bruchpunkt zur Biegung des ersten Stängelteils bewegbar. Auf diese Weise kann die Bastrinde zusammen mit den Holzbruchkörpern weiter gefördert werden und dieser Prozess idealerweise mit der Trennung der Holzbruchkörper von der Bastrinde kombiniert werden.

Anstatt den Bruchpunkt zusammen mit der Bastrinde zu einem Trennmittel zu bewegen, kann alternativ ein Trennmittel ausgehend von dem Bruchbereich zwischen Bastrinde und dem ersten Holzbruchkörper entlang des ersten Holzbruchkörpers bewegt werden, um die Bastrinde vom ersten Holzbruchkörper zu trennen. Auf diese Weise wird das Abziehen der Bastrinde vom Holzbruchkörper dahingehend vereinfacht, dass das Trennmittel das Teilungsgewebe zur Entlastung des Zugmittels in Längsrichtung auftrennen kann. Auf diese Weise ist es möglich, dass die Bastrinde sauber vom ersten geschnittenen Holzkörper abgetrennt wird. Beispielsweise kann die Zugkraft durch die Bewegung der Bastrinde am Bruchpunkt übertragen werden, sodass eine Relativbewegung zum Trennmittel das Abschälen der Bastrinde durch die physische Präsenz des Trennmittels am aufbrechenden Teilungsgewebe für eine saubere Trennung sorgt. Somit kann das Trennmittel auch ein feststehendes Element sein.

Alternativ zur Knick-Bruch-Isolierung kann der Bastpflanzenstängel vor der Anwendung der Zugkraft an einem ersten Ende in Richtung der Längsachse derart angeschnitten werden, dass der Bastpflanzenstängel endseitig in wenigstens zwei Hohlzylindersegmente geteilt wird. Dieser Vorgang kann als ein endseitiges Aufspleißen bezeichnet werden, wobei durch das Eintreten, beispielsweise einer Klinge oder einem anderen Schneideelement der Holzkörper derart auseinandergedrückt wird, dass der Holzkörper im endseitigen Bereich bricht. Anschließend kann mittels eines zusätzlichen Elements eine Verschiebekraft in das Teilungsgewebe eingeleitet werden, sodass sich die Bastrinde in radialer Richtung von der Längsachse des Holzkörpers entfernt, falls dies nicht bereits beim endseitigen Anschnitt erzielt wurde. Nunmehr ist es mit einem Zugelement, wie beispielsweise einem Rollen- oder Walzenpaar, möglich, die so isolierten Enden der Bastrinde zu greifen und die Bastrinde vom Holzkörper des restlichen Bastpflanzenstängels abzuziehen. Zum endseitigen Spleißen sind auch mehrere Schneidevorgänge denkbar oder alternativ Schneideelemente, beispielsweise mit einer Mehrzahl von Klingen oder rotierenden Scheibenmessern, die gleichzeitig stirnseitig und/oder seitlich in den Bastpflanzenstängel getrieben werden können. Vorteilhafterweise wird durch Anwendung der Zugkraft ein Teil der Bastrinde beginnend an einem der Hohlzylindersegmente vom Holzkörper getrennt, wobei die Anzahl der entstehenden Holzzylindersegmente von der Anzahl der Teilungsvorgänge oder Anzahl der Schneideelemente abhängt.

Eine Anpassung der Zugkraft sowie anderer Parameter ist bei Bastpflanzenstängeln in unterschiedlichen Stadien erforderlich, wie zum Beispiel bei erntefrischen, getrockneten, gerösteten oder wiederbefeuchteten Bastpflanzenstängeln. In jedem Fall wird eine entsprechende Zugkraft zu bestimmen sein, die es ermöglicht, das erfindungsgemäße Verfahren auszuführen.

Bei einer vorteilhaften Ausführungsform ist die Vorrichtung zur Isolierung von Bastrinde und Holzkörper aus Bastpflanzenstängeln, die ein erfindungsgemäßes Verfahren anwendet, eine stationäre oder eine mobile Maschine. Derartige Maschinen sind in der Lage, einzelne oder eine Vielzahl von Bastpflanzenstängeln in Form von Bündeln oder Ballen erfindungsgemäß zu verarbeiten. Dabei kommen zusätzliche Komponenten zum Einsatz, die beispielsweise die Ernte, die Beförderung, die Anordnung oder die Konditionierung der Bastpflanzenstängel realisieren.

Die erfindungsgemäße Vorrichtung zur Isolierung von Bastrinde und Holzkörper aus Bastpflanzenstängeln weist folgende Komponenten auf:
a. Mittel zur Teilung (Teilungsmittel) und Herstellung eines ersten und zweiten Stängelteils zur Durchschneidung des Holzkörpers entlang der Längsachse des Bastpflanzenstängels, so dass der erste Stängelteil einen ersten geschnittenen Holzkörper und der zweite Stängelteil einen zweiten geschnittenen Holzkörper aufweist,
b. Mittel zur Biegung (Biegemittel) wenigstens des ersten Stängelteils, bei dem die Bastrinde des ersten Stängelteils an einer gestauchten Stelle angeordnet ist und mit dem die Biegekraft so lange erhöht wird, bis der erste geschnittene Holzkörper auf einer der gestauchten Stelle gegenüberliegenden, gedehnten Stelle des Stängelteils von der Bastrinde weg, derart nach außen aufbricht, so dass aus dem ersten geschnittenen Holzkörper ein erster Holzbruchkörper und ein zweiter Holzbruchkörper gebildet werden,
c. Mittel zur Trennung (Trennmittel) der Bastrinde vom ersten und/oder zweiten Holzbruchkörper.

Vorteilhafterweise ist ein erstes Teilungsmittel ein Pflanzenstängelförderelement, insbesondere ein Walzenpaar oder Rollenpaar, und ein zweites Teilungsmittel eine Pflanzenstängelschneide zur Durchschneidung des Bastpflanzenstängels entlang der Längsachse des Bastpflanzenstängels. Die Positionierung der Schneide im Bezug zu den Pflanzenstängelförderelementen ist entscheidend, wenn es darum geht, den Bastpflanzenstängel möglichst mittig zu durchtrennen oder eine vielfache Teilung anzustreben.

Vorteilhafterweise ist ein erstes Biegemittel dazu vorgesehen, nach einem Bruch des ersten geschnittenen Holzkörpers beide durch den Bruch entstandenen Holzkörperbruchteile durch eine Einwirkung auf die Bastrinde in eine Bewegung zu bringen. Somit ist es möglich, mit der Bewegung des ersten Biegemittels sowohl den Bruch des ersten geschnittenen Holzkörpers herbeizuführen, als auch die Abtrennung zu ermöglichen, die ebenfalls durch eine Relativbewegung des ersten Biegemittels in einem bestimmten Winkel zur Stängelachse bewerkstelligt werden kann.

Vorteilhafterweise ist die Vorrichtung dazu vorgesehen, eine Vielzahl von Bastpflanzenstängeln oder Stängelteilen gleichzeitig zu bearbeiten. Dies kann die unterschiedlichsten Arbeitsschritte betreffen, wie zum Beispiel die Biegung, den Bruch und die Trennung der Bastrinde vom Holzkörper bzw. vom geschnittenen Holzkörper.

Bei einer vorteilhaften Ausführungsform ist die Vorrichtung dazu vorgesehen, vor dem Biegevorgang eine Vielzahl von Stängelteilen derart parallel zueinander auszurichten, dass deren Schnittflächen in die gleiche Richtung orientiert sind und ein erstes Biegemittel dazu vorgesehen ist, diese Vielzahl von Stängelteilen gleichzeitig zu biegen und damit gezielt zu brechen. Beispielsweise ist ein erstes Trennmittel ein Rollenpaar oder Walzenpaar. Ein Rollenpaar oder Walzenpaar ist zwar in erster Linie eine Fördervorrichtung, jedoch wirkt dessen Fördereffekt auf eine Trennung der Bastrinde vom Holzkörper hin, was das Rollen oder Walzenpaar zu einem Trennmittel macht. Als Trennmittel können auch Bolzen, Stangen oder Kanten verwendet werden, die zwischen die Bastrinde und den Holzkörper oder einen geschnittenen Holzkörper zu bringen sind.

Mittels des erfindungsgemäßen Verfahrens sind Produkte aus der Bastrinde (sowie daraus gewonnen Bastfasern und deren spezifischen Komponenten) und dem Holzkörper herstellbar. Diese Produkte werden nachfolgend Bastrindenprodukte und Bastfaserprodukte genannt.

Der durch das erfindungsgemäße Verfahren isolierte Ausgangsrohstoff Bastrinde ist in seinem Zellmaterial kaum beschädigt worden und weist auch eine erhebliche Länge auf. Damit eignen sich die Bastfasern als auch die Bastrinde selbst im Wesentlichen für alle Applikationen, für die typischerweise auch Kunststofffasern eingesetzt werden. Durch entsprechende Verfahren zur Einkürzung kann die Bastrinde in der gleichen Art eingesetzt werden wie bisherige Kurzfasern. Dabei weist die erfindungsgemäße Bastrinde deutlich bessere Materialeigenschaften auf und führt zu hochwertigeren Bastfaserprodukten wie z.B. insbesondere flächen- oder linienförmigen, textilen Gebilden aus natürlich endlicher oder durch entsprechende Prozesse endlos konfektionierter Bastrinde.

Vorteilhafterweise weist die erfindungsgemäß gewonnene Bastrinde oder die gewonnene Bastfaser einen Holzanteil von weniger als 1% auf und ist damit im Wesentlichen schäbenfrei. Folglich ist auch das Bastfaserprodukt als auch das Bastrindenprodukt schäbenfrei.

Bei einer vorteilhaften Ausführungsform ist eine Mehrzahl von Bastrindenstreifen in einem Gelege zueinander angeordnet. Dieses Gelege kann beispielsweise als Matte ausgebildet sein und einen besonders zugfesten Werkstoff bilden, der insbesondere im Automobilbau zur Verstärkung von flächigen Bauteilen eingesetzt werden kann.

Vorteilhafterweise sind die Bastrindenstreifen des Geleges miteinander verwebt oder vernäht. Bei einer Verwebung ist die Handhabbarkeit des Geleges beim Integrieren in eine Matrix oder beim Eingießen deutlich vorteilhafter. Außerdem ist das Gelege unempfindlicher gegen eine Deplatzierung von zueinander angeordneten Bastrinden. Zwei oder mehrere Lagen von Bastrinden können miteinander vernäht werden, wobei ebenfalls eine strukturell bedingte Stabilität generiert wird. Diese Malimotechnik wird bereits als textiltechnologisches Fertigungsverfahren eingesetzt.

Das Gelege kann ferner mit unidirektional angeordneten Bastrinden oder mit multidirektional angeordneten Bastrinden gebildet sein. Beides hat einen Einfluss auf die Gleichmäßigkeit und die Stabilität. Außerdem können Gelege dreidimensional oder zweidimensional ausgebildet sein. Gerade in Verbindung mit einer Matrix kann das Gelege schon die endgültige Form des herzustellenden Bauteils, zum Beispiel eine Fahrzugtür, ausbilden und mittels der Matrix fixiert werden.

Bei einer vorteilhaften Ausführungsform ist das Gelege als Bewehrung in einer Matrix eingebettet. Unter Matrix wird ein die Bastrinde oder die Bastfasern starr aufnehmender, ausgehärteter Stoff verstanden, der die Bastrinde beziehungsweise die Bastfasern durch Adhäsiv- oder Kohäsivkräfte bindet. Eine Matrix wird typischerweise gegossen und fixiert die Bastrinde oder Bastfasern in ihrer Position zueinander. Durch die Verwendung von Faserwerkstoffen haben Faser-Kunststoff-Verbunde normalerweise ein richtungsabhängiges Elastizitätsverhalten. Mit der Matrix ist ein Verbundwerkstoff gebildet, der als Zwischenprodukt weiterverarbeitet werden kann. Die Bastrinde oder die Bastfasen können ferner einer Vorbehandlung unterzogen werden, die die bessere Anbindung an die Matrix unterstützt. Eine Vorbehandlung könnte zum Teil oder ganz aus einer Reinigung der Bastrindenstreifen bestehen.

Vorteilhafterweise ist die Matrix eine Kunststoffmatrix, eine Harzmatrix, eine Matrix auf Pflanzenölbasis oder eine Matrix auf der Basis eines petrochemischen Rohstoffs. Duroplaste auf Pflanzenölbasis bilden eine vorteilhafte Matrix, die zwar eine Anbindung an die Bastrinde erfordert, aber biogen und zugleich stabil sind. Alternativ können auch bewährte Thermoplaste oder Harze als Matrix verwendet werden.

Vorteilhafterweise werden natürliche Matrixstoffe (auch Kittstoffe genannt), wie zum Beispiel Pektine oder Lignine, zur Herstellung einer natürlichen Matrix eingesetzt, sodass das Bastrindenprodukt komplett oder größten Teils biogen ist. Gerade bei einer solchen natürlichen Matrix lohnt sich als Vorbehandlung eine Reinigung der Bastrindenstreifen, um eine optimale Anbindung der Bastrindenstreifen an die natürliche Matrix sicherzustellen. Die Festigkeiten bekannter Kunstfasern basierter Verbundwerkstoffe werden durch den Verbundwerkstoff auf Bastrindenbasis um ein Vielfaches überschritten. Der Verbundwerkstoff auf Bastrindenbasis ist nur ein Beispiel eines Bastrindenproduktes.

Vorteilhafterweise haften noch Reste des Teilungsgewebes an den Bastfasern.

Dies stellt ein Herstellungsvorteil dar. Im Einzelfall und in Abhängigkeit von der Anwendung ist es durchaus tolerierbar, dass das Teilungsgewebe noch zum Teil an der Bastrinde anhaftet. Anhand des Teilungsgewebes, welches nach der Trennung austrocknet, ist erkennbar, dass es sich um Bastrinde handelt, die mit dem erfindungsgemäßen Verfahren isoliert wurde.

In manchen Fällen wird die Bastrinde nach der Abtrennung weiter behandelt und insbesondere mechanisch, chemisch, biologisch oder hydrothermisch aufgeschlossen. Vorteilhafterweise kann das Bastfaserprodukt ein Bastfaserverbundwerkstoff, ein Bastfasergelege oder ein Bastfasergarn sein. Bei einem Bastfasergarn sind diverse Prozessschritte nach der Isolierung der Bastrinde erforderlich, um das Bastfasergarn herzustellen.

Ein weiteres wertvolles Produkt ist das Bastpflanzenprodukt, das mittels des erfindungsgemäßen Verfahrens gewonnen wird, und aus den Holzkörpern gefertigt ist. Die Holzkörper der Bastpflanze bestehen aus einem äußerst stabilen und leichten, verholzten Gewebe, welche ähnlich wie Balsaholz zu verarbeiten und einzusetzen sind.

Weitere vorteilhafte Ausbildungen und bevorzugte Weiterbildungen der Erfindung sind den Figurenbeschreibungen und / oder den Unteransprüchen zu entnehmen

Im Folgenden wird die Erfindung anhand der in den Figuren dargestellten Ausführungsbeispiele näher beschrieben und erläutert.

### Kurze Beschreibung der Figuren

Es zeigen:
- Figur 1a: ein Bastpflanzenstängel im Querschnitt,
- Figur 1b: eine Durchtrennung eines Bastpflanzenstängels,
- Figur 2: ein erster Arbeitsschritt der Knick-Bruch-Isolierung,
- Figur 3: ein zweiter Arbeitsschritt der Knick-Bruch-Isolierung zur Biegung und Bruch des Stängelteils,
- Figur 4: ein dritter Arbeitsschritt zur Einleitung der Isolation,
- Figur 5: ein vierter Arbeitsschritt der Knick-Bruch-Isolierung,
- Figur 6: einen Beginn eines fünften Arbeitsschrittes der Knick-Bruch-Isolierung,
- Figur 7: eine Darstellung des fünften Arbeitsschrittes zu Figur 6,
- Figur 8: ein erster Arbeitsschritt eines zweiten Ausführungsbeispiels des Verfahrens zu Beginn des Knick-Bruches,
- Figur 9: ein zweiter Arbeitsschritt des zweiten Ausführungsbeispiels,
- Figur 10: ein dritter Arbeitsschritt des zweiten Ausführungsbeispiels,
- Figur 11: ein vierter Arbeitsschritt des zweiten Ausführungsbeispiels für den Übergang vom Knick-Bruch zur Isolation,
- Figur 12: ein fünfter Arbeitsschritt des zweiten Ausführungsbeispiels,
- Figur 13: eine dreidimensionale Ansicht einer endseitigen Stängelteilung eines dritten Ausführungsbeispiels des Verfahrens, und
- Figur 14: eine Trennung der Bastrinde gemäß dem dritten Ausführungsbeispiel zur Figur 13.

### Erläuterung der Figuren

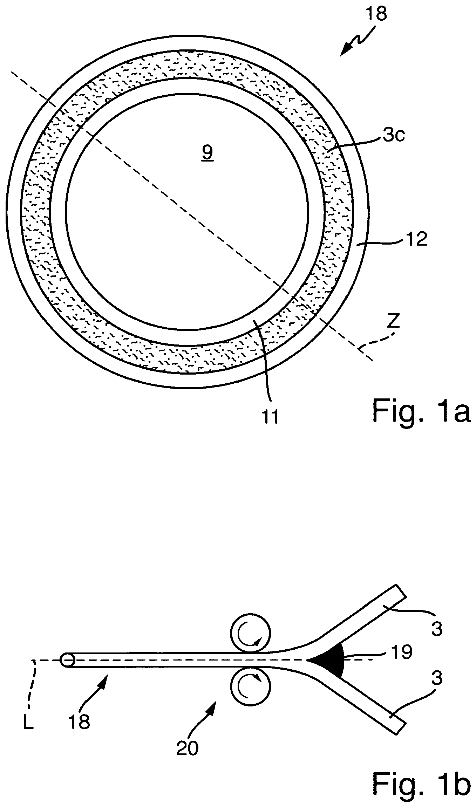

Figur 1a zeigt einen Bastpflanzenstängel im Querschnitt, wobei die Schnittfläche Z im Wesentlichen mittig durch den Holzkörper 9 verläuft. Der Holzkörper 9 ist über das Teilungsgewebe 11 mit der Bastrinde 3c verbunden. Man könnte auch sagen, dass der Holzkörper 9 aufgrund der Anordnung durch das Teilungsgewebe 11 von der Bastrinde 3c getrennt ist. Der Bastpflanzenstängel 18 wird außenseitig durch die Außenhaut 12 geschützt, die um die Bastrinde 3c herum angeordnet ist.

Figur 1b zeigt einen Bastpflanzenstängel 18, der durch das Walzenpaar 20 entlang der Längsachse L gefördert wird, um an der Pflanzenstängelschneide 19 in zwei Stängelteile 3 im Wesentlichen mittig geteilt zu werden. Die mittige Spaltung ist nicht zwingend, ist aber verfahrensbedingt von Vorteil, da beide Stängelteile 3 in der gleichen Weise behandelt werden können.

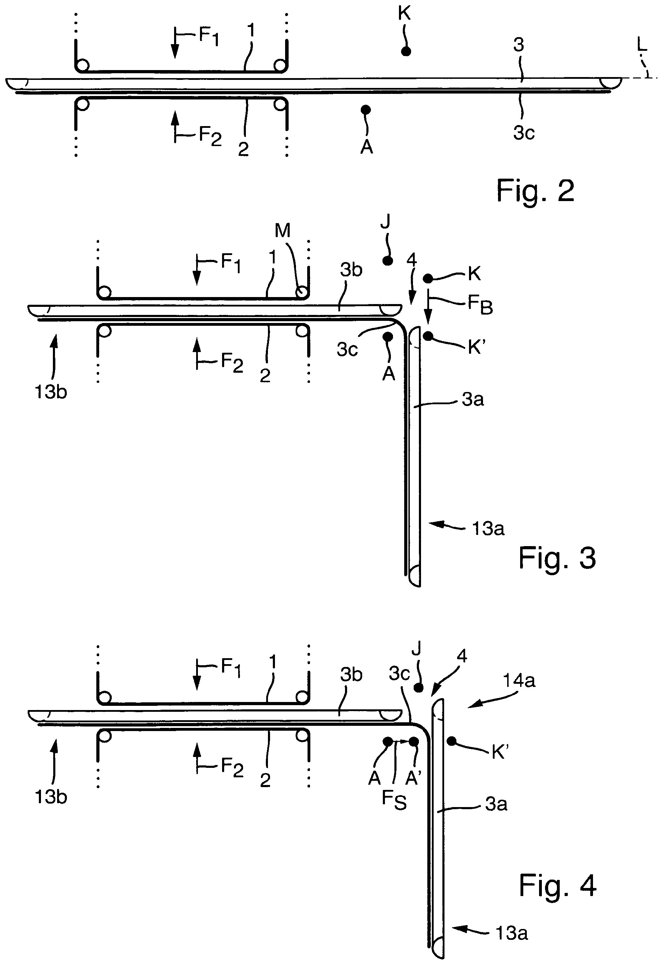

In den nachfolgenden Figuren 2 bis 14 wird auf die Außenhaut 12 kein Bezug genommen. Stattdessen wird vereinfachend leidglich von der Bastrinde 3c gesprochen.

Figur 2 zeigt einen der Stängelteile 3 aus Figur 1b der mittels einer oberen Fördereinrichtung 1 und einer unteren Fördereinrichtung 2, die jeweils mit der Haltekraft F1, beziehungsweise mit der Haltekraft F2, den Stängelteil 3 zwischen sich festhalten und gleichzeitig entlang der Längsachse des Stängelteils bewegen, sodass der Stängelteil 3 zwischen den Biegepunkten A und K angeordnet werden kann. Dabei stellt A einen inneren Biegepunkt und K einen äußeren Biegepunkt dar.

Figur 3 zeigt Biegung und Bruch des Stängelteils 3, wobei der bewegbare äußere Biegepunkt K senkrecht zur Längsachse L geführt wird, um an dem Biege- und Bruchpunkt A einen offenen Bruchbereich 4 hervorzurufen. Nach vollzogenem Bruch hat sich der äußere Biegepunkt K an die Position K' bewegt. Hierbei handelt es sich um eine Dreipunktbiegung, beziehungsweise um einen Dreipunktbruch, wobei die obere Fördereinrichtung 1 als äußerer Biegepunkt M fungiert. Somit hat sich der äußere Biegepunkt K gegenüber den Biegepunkten A, M relativ bewegt, wobei A und M statische Biegepunkte sein können. Alternativ ist es denkbar, dass sich zwei oder auch alle drei Biegepunkte A, K, M bewegen.

Am Bruchpunkt A liegt die unbeschädigte Bastrinde 3c an, in der die Bastfasern enthalten sind. Die nachfolgenden Figuren 4, 5, 6 und 7 zeigen die Trennung beider Stängelteile 3a, 3b von der unbeschädigten Bastrinde 3c.

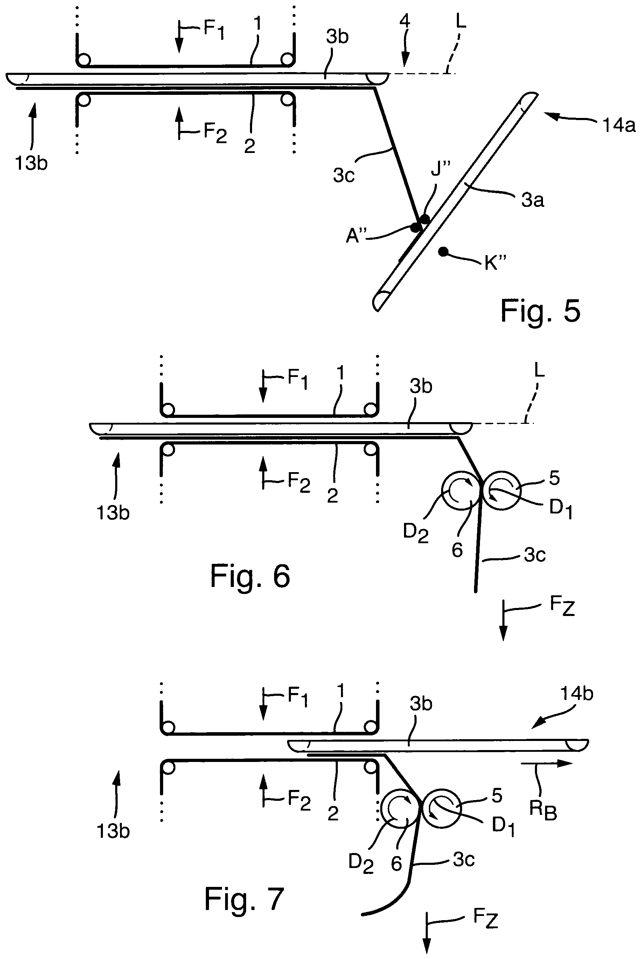

Figur 4 und 5: Die Abtrennung des ersten Holzbruchkörpers 3a wird nun dadurch gewährleistet, dass der vormals feste Biegepunkt A mittels der Kraft F_{S} in Längsrichtung des zweiten Holzbruchkörpers gefördert und gleichzeitig im Wesentlichen senkrecht zum ersten Holzbruchkörper bewegt wird. Somit trennt sich im Bruchbereich 4 am Ende 14a die Bastrinde 3c soweit ab, damit das Trennmittel J zwischen Bastrinde 3c und dem ersten Holzbruchkörper gebracht werden kann. Nunmehr können das Trennmittel J und die Biegepunkte A, K im Wesentlichen senkrecht zur Längsrichtung des zweiten Holzbruchkörpers bewegt werden, sodass eine Schälung der Bastrinde 3c eintritt und der erste Holzbruchkörper 3a von der Bruchstelle 4 entfernt wird. Dies kann bis zur vollständigen Trennung des ersten Holzbruchkörpers 3a von der Bastrinde 3c fortgesetzt werden.

Figur 6 und 7 zeigen die nachfolgende Trennung der Bastrinde 3c vom zweiten Holzbruchkörper 3b, indem ein Rollenpaar 5, 6 dazu verwendet wird, durch eine entgegengesetzte Drehung der Rollen 5 und 6 die Faserbastrinde 3c senkrecht zur Längsrichtung des zweiten Holzbruchkörpers zu bewegen. Auf diese Weise wird in Kombination mit den Fördereinrichtungen 1, 2 eine Bewegung des zweiten Holzbruchkörpers 3b in dessen Längsrichtung L veranlasst, sodass gleichzeitig die Abtrennung erfolgt.

Figur 7 zeigt die Abtrennung der Bastrinde 3c vom zweiten Holzbruchkörper 3b in einem fortgeschrittenen Stadium.

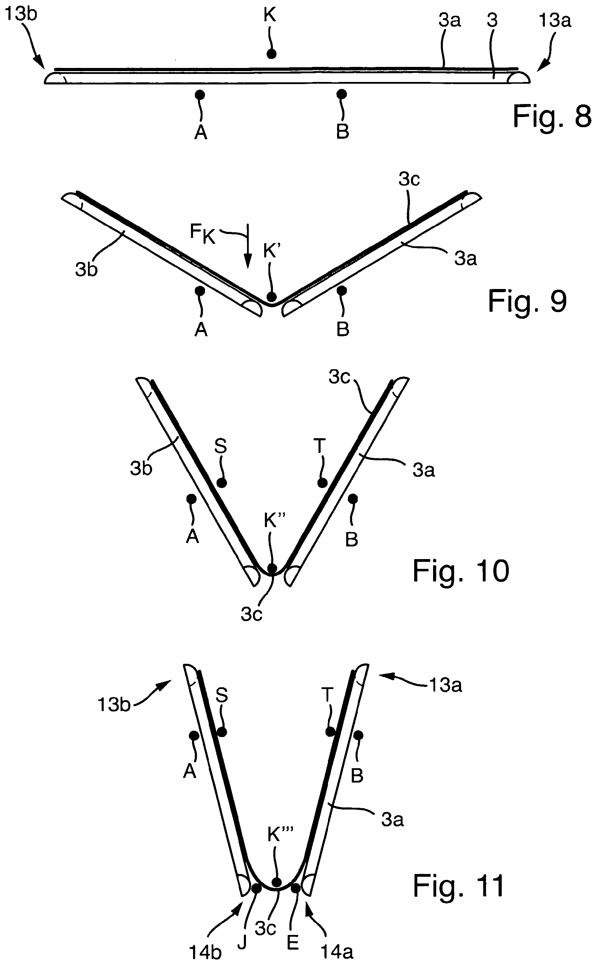

Figuren 8 bis 12 zeigen ein zweites Ausführungsbeispiel der erfindungsgemäßen Knick-Bruch-Isolierung in einer Dreipunktbiegung. Der innere Biegepunkt K bewegt sich relativ zu den äußeren Biegepunkten A und B. Dabei liegt der innere Biegepunkt K an der Bastrinde 3c an und die äußeren Biegepunkte A, B an der Schnittfläche des geschnittenen Holzkörpers. Die äußeren Biegepunkte A und B bewegen sich nicht, wobei der innere Biegepunkt K zwischen den Biegepunkten A, B durchläuft.

In einer neuen Position K' des Biegepunktes K, bei der Anwendung der Biegekraft F_{K}, findet der Bruch des Stängelteils 3 in die Holzbruchkörper 3a, 3b statt. Danach wird die Bastrinde 3c durch die Bewegung des Biege- und Bruchpunktes K zusammen mit den beiden Holzbruchkörpern 3a, 3b vorangetrieben, wobei durch die Haltepunkte S, T in Kombination mit den Biegepunkten A,B eine Führung der beiden Holzbruchkörper 3a, 3b erfolgt.

In einer neuen Position K'" ist die Bewegung soweit fortgesetzt, dass die Bastrinde 3c zwischen den Trennmitteln J, E platziert wird und gleichzeitig Trennmittel J, E zwischen die Bastrinde 3c sowie den ersten und zweiten Holzbruchkörper 3a, 3b in Position gebracht sind. Auf diese Weise wird durch eine einfache Fortsetzung der Biege- und Bruchbewegung unmittelbar die Isolierung der Bastrinde 3c realisiert.

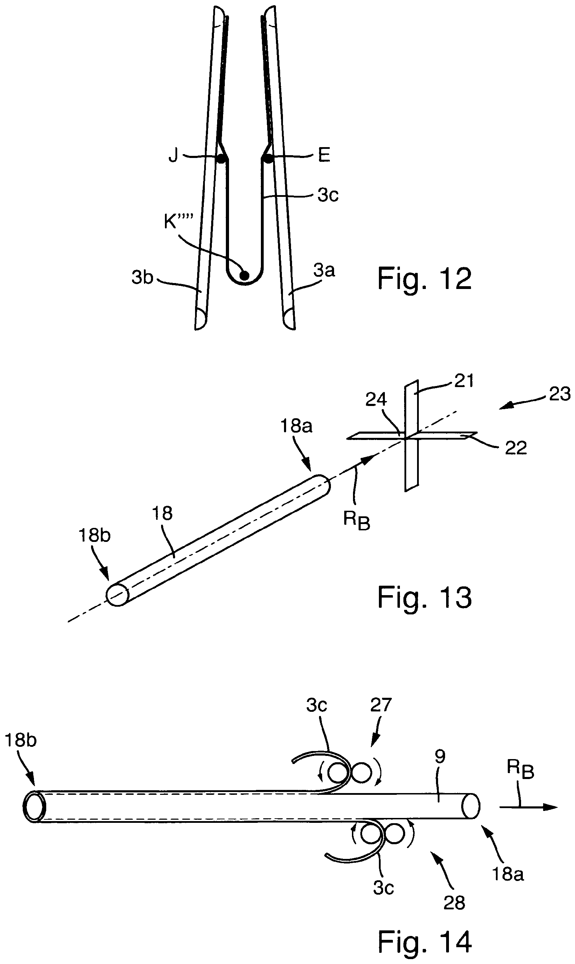

In Figur 12 ist die Trennungsbewegung in einem fortgeschrittenen Stadium dargestellt. Dabei ist der Bruch- und Biegepunkt K4"" soweit verschoben, dass die Bastrinde 3c nahezu hälftig von den Holzbruchkörpern 3a, 3b abgetrennt ist. Zur vollständigen Abtrennung wird die Bewegung des Biege- und Bruchpunktes K fortgesetzt.

Die Anordnungen zur Ausführung der Knick-Bruch-Isolierung in den Figuren 2 bis 12 sind in den gezeigten Biegepunkten A, B und Trennmitteln E, J und Haltepunkte S, T beispielsweise durch lange Stangen realisierbar, womit pro ausgeführter Biegung, beziehungsweise ausgeführtem Bruch, mehrere Stängelteile 3 in einer Vielzahl gebogen und gebrochen werden können. Dazu ist das Stängelteil 3 lediglich in einer Vielzahl auszuführen und parallel zueinander anzuordnen, wobei die Schnittflächen oder Spaltflächen zur gleichen Seite orientiert sein müssen.

Figur 13 zeigt eine alternative Ausführungsform des erfindungsgemäßen Verfahrens, bei dem der Bastpflanzenstängel 18 in Längsrichtung bewegt und beispielsweise durch ein Schneideelement 23 mit den Klingen 21, 22 in Punkt 24 angeschnitten und damit aufgespleißt werden kann. Bei diesem Vorgang wird der Holzkörper 9, und auch die Bastrinde 3c endseitig in vier Teile gespaltet.

Figur 14 zeigt das Abschälen der Bastrinde 3c von einem ungeteilten Bastpflanzenstängel 18, in dem Rollenpaare 27, 28 eingesetzt werden. Hierbei wird ganz automatisch durch das Einfangen und Abziehen der Bastrinde 3c eine Bewegung des Holzkörpers 9 in die Richtung R_{B} verursacht, in die auch der abgeschälte Holzkörper 9 ausgeworfen wird.

Zusammenfassend betrifft die Erfindung ein energieeffizientes Verfahren und eine Vorrichtung zur Isolierung von Bastrinde 3c und Holzkörper 9 aus einem Bastpflanzenstängel 18, beziehungsweise aus einer Vielzahl von Bastpflanzenstängeln 18, sowie isolierte Bastrinde 3c und Holzkörper 9. Diese sowie daraus erzeugte Produkte weisen eine höhere Qualität auf als solche aus Bastfasern oder Schäben nach dem Stand der Technik. Dazu wird vorgeschlagen, dass die Bastrinde 3c mittels einer Zugkraft F_{Z} entlang der in der Bastrinde 3c verlaufenden Bastfasern vom Holzkörper 9 oder zumindest von einem Bestandteil 3a, 3b des Holzkörpers 9 entlang des Teilungsgewebes 11 getrennt wird. Auf diese Weise entstehen Synergien sowohl bei der Isolierung, als auch bei der Verwertung des Rohstoffes Bastrinde 3c und der Holzkörper 9. Entsprechend weisen neben derartigen Bastrindenprodukten auch die zugehörigen Bastfaserprodukte vorteilige Merkmale auf.

### Bezugszeichenliste

- A: Biegepunkt
- B: Biegepunkt
- D₁: Drehrichtung
- D₂: entgegengesetzte Drehrichtung
- E: Trennmittel
- D₁: Haltekraft
- F₂: Haltekraft
- F_{B}: Biegekraft
- F_{K}: Biegekraft
- F_{S}: Kraft in Längsrichtung
- F_{Z}: Zugkraft
- J: Trennmittel
- K: Brechpunkt, Biegepunkt
- L: Längsachse
- M: Biegepunkt
- R_{B}: Bewegungsrichtung
- S: Haltepunkt
- T: Haltepunkt
- Z: Schnittfläche
- 1: Förderband oben
- 2: Förderband unten
- 3: Stängelteil
- 3a: erster Holzbruchkörper
- 3b: zweiter Holzbruchkörper
- 3c: Bastrinde
- 4: Bruchbereich
- 5: Rolle
- 6: Rolle
- 9: Holzkörper
- 11: Teilungsgewebe
- 12: Außenhaut
- 13a: Ende
- 13b: Ende
- 14a: Ende
- 14b: Ende
- 18: Bastpflanzenstängel
- 18a: erstes Ende
- 18b: zweites Ende
- 19: Stängelschneide
- 20: Walzenpaar
- 21,22: Klingen
- 23: Schneidepunkt
- 24: Punkt
- 27: Rollenpaar
- 28: Rollenpaar

## Patentansprüche

1. Verfahren zur Isolierung von Bastrinde (3c) und Holzkörper (9) aus einem Bastpflanzenstängel (18), wobei der Bastpflanzenstängel (18) in seinem Inneren aus einem im Wesentlichen zylindrischen Holzkörper (9) gebildet ist und der Holzkörper (9) radial von einer Bastfasern enthaltenden Bastrinde (3c) umfasst wird und über ein Teilungsgewebe (11) mit der Bastrinde (3c) verbunden ist, mit den Verfahrensschritten:
a. Eine Herstellung eines ersten und zweiten Stängelteils (3), mittels Stängelteilung des Bastpflanzenstängels (18) entlang der Längsachse (L), wobei der erste Stängelteil (3) einen ersten geschnittenen Holzkörper und der zweite Stängelteil einen zweiten geschnittenen Holzkörper aufweist,
**gekennzeichnet durch**
b. eine Biegung wenigstens des ersten Stängelteils (3), wobei das erste Stängelteil (3) so angeordnet ist, dass die Stauchung auf der Bastrindenseite erfolgt und die Biegekraft (Fä) so lange erhöht wird, bis der erste geschnittene Holzkörper auf einer der gestauchten Stelle gegenüberliegenden, gedehnten Stelle des ersten Stängelteils (3) von der Bastrinde (3c) weg, derart nach außen aufbricht, dass aus dem ersten geschnittenen Holzkörper ein erster Holzbruchkörper (3a) und ein zweiter Holzbruchkörper (3b) gebildet werden, und **durch**
c. eine Trennung der Bastrinde (3c) vom ersten Holzbruchkörper (3a), wobei die Bastrinde (3c) mittels einer Zugkraft (F_{Z}) vom ersten Holzbruchkörper (3a) **durch** die Teilung des Teilungsgewebes (11) getrennt wird, wobei ein Trennmittel (E,J) zwischen die Bastrinde (3c) und den ersten Holzbruchkörper (3a) des Holzkörpers (9) eingebracht wird.

2. Verfahren nach Anspruch 1, wobei die Zugkraft (F_{Z}) derart auf die Bastrinde (3c) ausgeübt wird, dass die Bastrinde (3c) einen Winkel zur Bewegungsrichtung (R_{B}) oder zur Längsachse (L) des ersten Holzbruchkörpers (3a) aufspannt, von dem die Bastrinde (3c) abgetrennt wird.

3. Verfahren nach Anspruch 1, wobei die Biegung des ersten Stängelteils (3) eine Dreipunktbiegung oder eine Vierpunktbiegung ist.

4. Verfahren nach Anspruch 3, wobei der geschnittene Holzkörper des ersten Stängelteils (3) wenigstens zwei äußere Biegepunkte (M, K) und die Bastrinde (3c) des ersten Stängelteils (3) wenigstens einen inneren Biegepunkt (A), insbesondere zwei innere Biegepunkte, berührt.

5. Verfahren nach einem der Ansprüche 3 oder 4, wobei wenigstens ein innerer Biegepunkt oder wenigstens ein äußerer Biegepunkt (K) eine Relativbewegung zu einem anderen inneren oder äußeren Biegepunkt (A, M) ausführt.

6. Verfahren nach Anspruch 3 bis 5, wobei der wenigstens eine innere Biegepunkt (A) ein Bruchpunkt (A) ist.

7. Verfahren nach einem der Ansprüche 3 bis 6, wobei das Trennmittel (J) ausgehend von einem Bruchbereich (4) zwischen Bastrinde (3c) und dem ersten Holzbruchkörper (3a) so bewegt wird, dass die Bastrinde (3c) vom ersten Holzbruchkörper (3a) zu trennen ist, wobei der noch nicht getrennte Stängelteil in seiner Beweglichkeit eingeschränkt ist.

8. Verfahren nach einem der Ansprüche 1 oder 2, wobei der Bastpflanzenstängel (18) vor der Anwendung der Zugkraft (F_{Z}) an einem ersten Ende (18a), in Richtung der Längsachse (L) derart angeschnitten wird, dass der Bastpflanzenstängel (18) endseitig in wenigstens zwei Hohlzylindersegmente geteilt wird.

9. Verfahren nach Anspruch 8, wobei ein Teil der Bastrinde (3c) beginnend an einem Ende der Hohlzylindersegmente durch Anwendung der Zugkraft (F_{Z}) vom Holzkörper (9) getrennt wird.

10. Vorrichtung, insbesondere Maschine, zur Isolierung von Bastrinde (3c) und Holzkörper (9) aus einem Bastpflanzenstängel (18), wobei der Bastpflanzenstängel (18) in seinem Inneren aus einem im Wesentlichen zylindrischen Holzkörper (9) gebildet ist, der Holzkörper (9) radial von einer Bastfasern enthaltenden Bastrinde (3c) umfasst wird und über ein Teilungsgewebe (11) mit der Bastrinde (3c) verbunden ist und die Vorrichtung ein Trennmittel (E,J), ein Spaltmittel (19, 20) und ein Zugmittel (5,6) aufweist, wobei
a. das Spaltmittel (19, 20) in einem ersten Schritt zur Herstellung eines ersten und zweiten Stängelteils (3), mittels einer Stängelteilung des Bastpflanzenstängels (18) entlang der Längsachse (L) des Bastpflanzenstängels (18) vorgesehen ist und der erste Stängelteil (3) einen ersten geschnittenen Holzkörper und der zweite Stängelteil einen zweiten geschnittenen Holzkörper aufweist,
**gekennzeichnet durch**
b. Biegemittel (A, B, K, M) zur Biegung wenigstens des ersten Stängelteils (3) in einem zweiten Schritt, wobei die Bastrinde (3c) an einer gestauchten Seite des ersten Stängelteils (3) angeordnet und die Biegekraft (F_{B}) derart veränderbar ist, dass der erste geschnittene Holzkörper auf einer der gestauchten Stelle gegenüberliegenden, gedehnten Stelle des Stängelteils (3) von der Bastrinde (3c) weg, nach außen in einen ersten und zweiten Holzbruchkörper (3a,3b) aufbricht, und
c. Zugmittel (5,6) zur Trennung der Bastrinde (3c) vom gebildeten ersten und/oder zweiten Holzbruchkörper (3a, 3b) in einem dritten Schritt vorgesehen sind, wobei mittels einer Zugkraft (F_{Z}) die Bastrinde (3c) von dem ersten Holzbruchkörper (3a) **durch** die Teilung des Teilungsgewebes (11) trennbar ist und wobei das Trennmittel (E,J) zwischen die Bastrinde (3c) und den ersten Holzbruchkörper (3a) des Holzkörpers (9) eingebracht wird.

11. Vorrichtung nach Anspruch 10, wobei wenigstens ein erstes Biegemittel (A) dazu vorgesehen ist, nach einem Bruch des ersten geschnittenen Holzkörpers (3) beide, durch den Bruch entstandenen Holzbruchkörper (3a,3b) durch eine Einwirkung auf die Bastrinde (3c) in eine Relativbewegung zu Trennmitteln (E,J) zu bringen.

12. Vorrichtung nach den Ansprüchen 10 bis 11, wobei eine Vielzahl von Bastpflanzenstängeln (18) oder Stängelteilen (3) gleichzeitig bearbeitet werden.

13. Bastrindenprodukt, insbesondere ein Materialverbund, mit Bastrinde (3c) wobei die Bastrinde (3c) nach einem Verfahren nach Anspruch 1 bis 9 oder mittels einer Vorrichtung nach den Ansprüchen 10 bis 12 hergestellt wurde.

14. Bastfaserprodukt, insbesondere ein Materialverbund, mit aus einer Bastrinde (3c) gewonnenen Bastfasern, wobei die Bastrinde (3c) nach einem Verfahren nach Ansprüchen 1 bis 9 oder mittels einer Vorrichtung nach den Ansprüchen 10 bis 12 hergestellt wurde.

15. Bastpflanzenprodukt, gefertigt aus Holzbruchkörpern (3a, 3b) aus einem Verfahren nach Anspruch 1, hergestellt mittels einer Vorrichtung nach Anspruch 10 gewonnenen Holzbruchkörpern (3a, 3b) und/oder gefertigt aus im Wesentlichen zylindrischen Holzkörpern (9) aus einem Verfahren nach Anspruch 8.

## Claims

1. Method for isolating bast bark (3c) and wood body (9) from a bast plant stem (18), wherein in the interior thereof the bast plant stem (18) is composed of a substantially cylindrical wood body (9), and the wood body (9) is covered radially by a bast bark (3c) containing bast fibres and is connected to the bast bark (3c) by meristem tissue (11), comprising the method steps:
a. producing a first and second stem part (3) by dividing the bast plant stem (18) along the longitudinal axis (L), wherein the first stem part (3) comprises a first cut wood body and the second stem part comprises a second cut wood body,
**characterized by**
b. a bending at least of the first stem part (3), wherein the first stem part (3) is arranged so that the compression takes place on the bast bark side and the bending force (F_{B}) is increased until the first cut wood body at a stretched point of the first stem part (3) located opposite to the compressed point, breaks open outwardly away from the bast bark (3c) in such a manner that a first wood breaking body (3a) and a second wood breaking body (3b) are formed from the first cut wood body and
c. by a separation of the bast bark (3c) from the first wood breaking body (3a), wherein the bast bark (3c) is separated from the first wood breaking body (3a) by the division of the meristem tissue (11) by means of a tensile force (F), wherein a separating mean (E, J) is introduced between the bast bark (3c) and the first wood breaking body (3a) of the wood body (9).

2. The method according to claim 1, wherein the tensile force (F_{Z}) is exerted in such a manner on the bast bark (3c) that the bast bark (3c) spans an angle with respect to the movement direction (R_{B}) or with respect to the longitudinal axis (L) of the first wood breaking body (3a), from which the bast bark (3c) is separated.

3. The method according to claim 1, wherein the bending of the first stem part (3) is a three-point bending or a four-point bending.

4. The method according to claim 3, wherein the cut wood body of the first stem part (3) touches at least two outer bending points (M, K) and the bast bark (3c) of the first stem part (3) touches at least one inner bending point (A), in particular two inner bending points.

5. The method according to any one of claims 3 or 4, wherein at least one inner bending point or at least one outer bending point (K) executes a relative movement with respect to one other inner or outer bending point (A, M).

6. The method according to claim 3 to 5, wherein the at least one inner bending point (A) is a breakpoint (A).

7. The method according to any one of claims 3 to 6, wherein the separating mean (J) starting from a breakage area (4) between bast bark (3c) and the first wood breaking body (3a) is moved so that the bast bark (3c) is to be separated from the first wood breaking body (3a), wherein the not yet separated stem part is restricted in its movability.

8. The method according to any one of claims 1 or 2, wherein, before applying the tensile force (F_{Z}) to a first end (18a), the bast plant stem (18) is cut in such a manner in the direction of the longitudinal axis (L) that the bast plant stem (18) is divided at the end into at least two hollow cylinder segments.

9. The method according to claim 8, wherein a part of the bast bark (3c), beginning at one end of the hollow cylinder segment is separated from the wood body (9) by applying the tensile force (F_{Z}).

10. Apparatus, in particular a machine, for isolating bast bark (3c) and wood body (9) from a bast plant stem (18), wherein in the interior thereof the bast plant stem (18) is composed of a substantially cylindrical wood body (9), the wood body (9) is covered radially by a bast bark (3c) containing bast fibres, and is connected to the bast bark (3c) by meristem tissue (11) and the apparatus has a separating mean (E, J), a dividing means (19, 20) and traction means (5, 6), wherein
a. the dividing means (19, 20) is provided in a first step for production of a first and second stem part (3) by means of a stem division of the bast plant stem (18) along the longitudinal axis (L) of the bast plant stem (18) and the first stem part (3) comprises a first cut wood body and the second stem part comprises a second cut wood body,
**characterized by**
b. bending means (A, B, K, M) for bending at least the first stem part (3) in a second step, wherein the bast bark (3c) is arranged on a compressed side of the first stem part (3) and the bending force (F_{B}) can be varied in such a manner that the first cut wood body, at a stretched point of the stem part (3) located opposite the compressed point, breaks open away from the bast bark (3c) outwardly into a first and second wood breaking body (3a, 3b), and
c. traction means (5, 6) for separating the bast bark (3c) from the formed first and/or second wood breaking body (3a, 3b) in a third step, wherein by means of a tensile force (F_{Z}), the bast bark (3c) can be separated from the first wood breaking body (3a) by the division of the meristem tissue (11) and wherein the separating mean (E, J) is introduced between the bast bark (3c) and the first wood breaking body (3a) of the wood body (9).

11. The apparatus according to claim 10, wherein at least a first bending means (A) is provided to bring, after a breaking of the first cut wood body (3), both of the wood breaking bodies (3a, 3b) produced by the breaking into a relative movement with respect to the separating mean (E, J) by acting on the bast bark (3c).

12. The apparatus according to claims 10 to 11, wherein a plurality of bast plant stems (18) or stem parts (3) are processed simultaneously.

13. Bast bark product, in particular a material composite, comprising bast bark (3c), wherein the bast bark (3c) was produced by a method according to claims 1 to 9 or by means of an apparatus according to claims 10 to 12.

14. Bast fibre product, in particular a material composite comprising bast fibres obtained from a bast bark (3c), wherein the bast bark (3c) was produced by a method according to claims 1 to 9 or by means of an apparatus according to claims 10 to 12.

15. Bast plant product, produced from wood breaking bodies (3a, 3b) from a method according to claim 1, produced by means of wood breaking bodies (3a, 3b) obtained by means of an apparatus according to claim 10 and/or fabricated from substantially cylindrical wood bodies (9) from a method according to claim 8.

## Revendications

1. Procédé permettant d'isoler une écorce libérienne (3c) et un corps en bois (9) d'une tige de plante libérienne (18), en son intérieur, la tige de plante libérienne (18) étant formée d'un corps en bois (9) sensiblement cylindrique et le corps en bois (9) étant entouré en direction radiale par une écorce libérienne (3c) contenant des fibres libériennes et étant relié avec l'écorce libérienne (3c) par l'intermédiaire d'un tissu diviseur (11), présentant les étapes de procédé :
a. Une création d'une première et d'une deuxième parties de tige (3), par division de tige de la tige de plante libérienne (18) le long de l'axe longitudinal (L), la première partie de tige (3) comportant un premier corps en bois coupé et la deuxième partie de tige comportant un deuxième corps en bois coupé,
**caractérisé par**
b. un fléchissement d'au moins la première partie de tige (3), la première partie de tige (3) étant placée de telle sorte que le refoulement s'effectue sur le côté de l'écorce libérienne et que l'effort de flexion (F_{B}) soit augmenté jusqu'à ce qu'en un endroit étiré de la première partie de tige (3) qui est opposé à l'endroit refoulé, le premier corps en bois coupé se rompe en s'éloignant de l'écorce libérienne (3c), de sorte à former à partir du premier corps en bois coupé un premier fragment (3a) de corps en bois et un deuxième fragment (3b) de corps en bois et par
c. une séparation de l'écorce libérienne (3c) du premier fragment (3a) de corps en bois, l'écorce libérienne (3c) étant séparée à l'aide d'une force de traction (F_{Z}) du premier fragment (3a) de corps en bois par la division du tissu diviseur (11), un moyen de séparation (E, J) étant introduit entre l'écorce libérienne (3c) et le premier fragment (3a) de corps en bois du corps en bois (9).

2. Procédé selon la revendication 1, la force de traction (Fz) étant exercée sur l'écorce libérienne (3c) de telle sorte que l'écorce libérienne (3c) couvre un angle par rapport à la direction de déplacement (R_{B}) ou par rapport à l'axe longitudinal (L) du premier fragment (3a) de corps en bois duquel l'écorce libérienne (3c) est sectionnée.

3. Procédé selon la revendication 1, la flexion de la première partie de tige (3) étant une flexion en trois points ou une flexion en quatre points.

4. Procédé selon la revendication 3, le corps en bois coupé de la première partie de tige (3) étant en contact avec au moins deux points de flexion (M, K) extérieurs et l'écorce libérienne (3c) de la première partie de tige (3) étant en contact avec au moins un point de flexion (A) intérieur, notamment deux points de flexion intérieurs.

5. Procédé selon l'une quelconque des revendications 3 ou 4, au moins un point de flexion intérieur ou au moins un point de flexion extérieur (K) réalisant un déplacement relatif par rapport à un autre point de flexion (A, M) intérieur ou extérieur.

6. Procédé selon la revendication 3 à 5, l'au moins un point de flexion (A) intérieur étant un point de rupture (A).

7. Procédé selon l'une quelconque des revendications 3 à 6, le moyen de séparation (J) étant déplacé à partir d'une zone de rupture (4) entre l'écorce libérienne (3c) et le premier fragment (3a) de corps en bois de telle sorte que l'écorce libérienne (3c) doive être séparée du premier fragment (3a) de corps en bois, la partie de tige qui n'est pas encore séparée étant restreinte dans sa mobilité.

8. Procédé selon l'une quelconque des revendications 1 ou 2, avant l'application de la force de traction (F), la tige de plante libérienne (18) étant entaillée sur une première extrémité (18a) en direction de l'axe longitudinal (L), de telle sorte que la tige de plante libérienne (18) soit divisée côté extrémité en au moins deux segments de cylindre creux.

9. Procédé selon la revendication 8, une partie de l'écorce libérienne (3c) commençant sur une extrémité des segments de cylindre creux étant séparée du corps en bois (9) par l'application de la force de traction (F_{z}).

10. Dispositif, notamment machine permettant d'isoler une écorce libérienne (3c) et un corps en bois (9) d'une tige de plante libérienne (18), en son intérieur, la tige de plante libérienne (18) étant formée d'un corps en bois (9) sensiblement cylindrique, le corps en bois (9) étant entouré en direction radiale par une écorce libérienne (3c) contenant des fibres libériennes et étant relié avec l'écorce libérienne (3c) par l'intermédiaire d'un tissu diviseur (11) et le dispositif comportant un moyen de séparation (E, J), un moyen de scission (19, 20) et un moyen de traction (5, 6)
a. le moyen de scission (19, 20) étant prévu dans une première étape destinée à créer une première et une deuxième parties de tige (3), au moyen d'une division de tige de la tige de plante libérienne (18) le long de l'axe longitudinal (L) de la tige de plante libérienne (18) et la première partie de tige (3) comportant un premier corps en bois coupé et la deuxième partie de tige comportant un deuxième corps en bois coupé,
**caractérisé par**
b. des moyens de flexion (A, B, K, M) destinés à infléchir au moins la première partie de tige (3) dans une deuxième étape, l'écorce libérienne (3c) étant placée sur une face refoulée de la première partie de tige (3) et la force de flexion (F_{B}) étant variable de telle sorte qu'en un endroit étiré de la partie de tige (3) qui est opposé à l'endroit refoulé, le premier corps en bois coupé se rompe en s'éloignant de l'écorce libérienne (3c) vers l'extérieur, en un premier et un deuxième fragments (3a, 3b) de corps en bois, et
c. des moyens de traction (5, 6) destinés à séparer l'écorce libérienne (3c) du premier et/ou deuxième fragment (3a, 3b) de corps en bois créé, dans une troisième étape sont prévus, à l'aide d'une force de traction (F_{z}), l'écorce libérienne (3c) étant séparable du premier fragment (3a) de corps en bois par la division du tissu diviseur (11) et le moyen de séparation (E, J) étant introduit entre l'écorce libérienne (3c) et le premier fragment (3a) de corps en bois du corps en bois (9).

11. Dispositif selon la revendication 10, au moins un premier moyen de flexion (A) étant prévu, par une action sur l'écorce libérienne (3c) pour amener après une rupture du corps en bois (3) coupé les deux fragments (3a, 3b) de corps en bois issus de la rupture dans un déplacement relatif par rapport à des moyens de séparation (E, J).

12. Dispositif selon les revendications 10 ou 11, une pluralité de tiges de plantes libériennes (18) ou de parties de tiges (3) étant traitées en même temps.

13. Produit à base d'écorce libérienne, notamment une matière composite, avec de l'écorce libérienne (3c), l'écorce libérienne (3c) ayant été fabriquée d'après un procédé selon la revendication 1 à 9 ou à l'aide d'un dispositif selon les revendications 10 bis 12.

14. Produit à base de fibre libérienne, notamment une matière composite, avec des fibres libériennes recueillies à partir d'une écorce libérienne (3c), l'écorce libérienne (3c) ayant été fabriquée d'après un procédé selon la revendication 1 à 9 ou à l'aide d'un dispositif selon les revendications 10 bis 12.

15. Produit à base de plante libérienne, fabriqué à partir de fragments (3a, 3b) de corps en bois issus d'un procédé selon la revendication 1, fabriqué à l'aide de fragments (3a, 3b) de corps en bois recueillis à l'aide d'un dispositif selon la revendication 10 et/ou fabriqué à partir de corps en bois (9) sensiblement cylindriques, issus d'un procédé selon la revendication 8.
